# EUROPEAN PATENT APPLICATION

(11) **EP 4 252 670 A1**
(43) Date of publication of application: **04.10.2023**
(21) Application number: 22167640.6
(22) Date of filing: 11.04.2022
(51) Int. Cl.: A61B 8/08, A61B 8/00

(54) **METHODS AND SYSTEMS FOR ULTRASOUND-BASED STRUCTURE LOCALIZATION USING IMAGE AND USER INPUTS**

(30) Priority: 31.03.2022 US 202263325660 P
(71) Applicant: Koninklijke Philips N.V., 5656 AG Eindhoven (NL)
(72) Inventor: ZHENG, Mingxin, Eindhoven (NL); CHEN, Alvin, Eindhoven (NL); BHARAT, Shyam, Eindhoven (NL); JIANG, Baichuan, Eindhoven (NL)
(74) Representative: Philips Intellectual Property & Standards

(57) **Abstract**

A method (100) for providing localization information using an ultrasound system (200), comprising: receiving (140) a plurality of ultrasound images for a first imaged region; receiving (150), via a user interface, a user input comprising location information about the first imaged region; processing (160), using a trained localization algorithm, both: (i) one or more of the received plurality of ultrasound images and (ii) the received user input to generate a localization report for the first imaged region, the localization report comprising one or more of a coordinate, a contour, and a structure identification; and providing (170), via a user interface, the localization report to a user.

## Description

### FIELD OF THE INVENTION

The present disclosure is directed generally to methods and systems for localizing anatomical structures using an ultrasound system.

### BACKGROUND OF THE INVENTION

Compared to other imaging modalities such as fluoroscopy, computerized tomography (CT), and magnetic resonance imaging (MRI), ultrasound offers a low-cost, radiation-free, and real-time means to assess vessel morphology, topology, and blood flow. Similar advantages for other ultrasound-based imaging procedures are well-described in the clinical literature.

Today, however, clinical expertise and specialized training are needed to identify anatomical structures-of-interest, detect regions of disease, and obtain accurate measurements during an ultrasound scan. For example, in the case of peripheral arterial disease (PAD), small extremity arteries are particularly difficult to measure. Experienced sonographers often repeatedly switch between B-mode and color Doppler modes during a scan. For example, they may scan back-and-forth while visually tracking the arteries through complex anatomy. They may add markings on the screen, or take single-frame snapshots, to aid in visual tracking and to remember specific locations and landmarks. All of these steps require skill; losing sight of the target during a scan can result in a cumbersome workflow, the need for repeated scans, lengthy procedure times, and reduced procedure outcomes.

Existing methods for landmark or anatomical feature identification are based on either user experience or automated algorithms. As described above, experienced sonographers identify landmarks or anatomical features when viewing ultrasound imaging in real-time or via a recording, but this process is onerous, time-consuming, and fallible, especially with less experience. Algorithmic identification of landmarks or anatomical features are based on trained algorithms that receive and analyze one or more ultrasound images for identification. However, algorithmic identification is also an error-prone method.

### SUMMARY OF THE INVENTION

Accordingly, there is a continued need for methods and systems for improved localization of anatomical structures using an ultrasound system.

The present disclosure is directed to inventive methods and systems for analysis of ultrasound imaging. Various embodiments and implementations herein are directed to an ultrasound analysis system comprising an ultrasound device configured to obtain or otherwise receive a plurality of ultrasound images for a first imaged region. The system also receives, via a user interface, a user input comprising location information about the first imaged region. A trained localization algorithm of the system processes both: (i) one or more of the received plurality of ultrasound images; and (ii) the received user input to generate a localization report for the first imaged region, the localization report comprising one or more of a coordinate, a contour, and a structure identification. The system them provides the localization report to a user via a user interface.

Generally in one aspect, a method for providing localization information using an ultrasound system is provided. The method includes: (i) receiving a plurality of ultrasound images for a first imaged region; (ii) receiving an input comprising location information about the first imaged region; (iii) processing, using a trained localization algorithm, both: (1) one or more of the received plurality of ultrasound images and (2) the received input to generate a localization report for the first imaged region, the localization report comprising one or more of a coordinate, a contour, and a structure identification; and (iv) providing, via a user interface, the localization report to a user.

According to an embodiment, the method further includes receiving ultrasound scan information for a present or future scan; and directing, based on the received ultrasound scan information, the user to obtain the plurality of ultrasound images for the first imaged region.

According to an embodiment, the plurality of ultrasound images are received and processed in real time.

According to an embodiment, the trained localization algorithm is a segmentation algorithm or an object detection algorithm.

According to an embodiment, the localization report further comprises a confidence score generated by the trained localization algorithm.

According to an embodiment, the input is derived from the user's interaction with the ultrasound system while the user obtains the plurality of ultrasound images for the first imaged region.

According to an embodiment, the input is derived from one or more ultrasound scans or ultrasound images obtained or received before the received plurality of ultrasound images.

According to an embodiment, the input is an identification of a structure or feature within the first imaged region.

According to an embodiment, the method further includes: receiving, from the user via the user interface in response to the provided localization report, modifying input about the first imaged region; processing, using the trained localization algorithm, both: (i) one or more of the received plurality of ultrasound images and (ii) the received modifying input to generate a modified localization report for the first imaged region; and providing, via a user interface, the modified localization report to the user.

According to another aspect is an ultrasound system configured to provide localization information. The system includes: a plurality of ultrasound images for a first imaged region; a user interface configured to receive a user input comprising location information about the first imaged region; a trained localization algorithm; and a processor configured to process, using the trained localization algorithm both: (i) one or more of the received plurality of ultrasound images and (ii) the received user input to generate a localization report for the first imaged region; and direct the user interface to provide the localization report to a user.

According to another aspect is a non-transitory computer readable storage medium having computer readable program code embodied therein for causing an ultrasound system to provide localization information by: receiving a plurality of ultrasound images for a first imaged region; receiving an input comprising location information about the first imaged region; processing, using a trained localization algorithm, both: (i) one or more of the received plurality of ultrasound images and (ii) the received input to generate a localization report for the first imaged region, the localization report comprising one or more of a coordinate, a contour, and a structure identification; and providing, via a user interface, the localization report to a user.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig. 1 is a flowchart of a method for providing localization information using an ultrasound system, in accordance with an embodiment.
Fig. 2 is a schematic representation of an ultrasound analysis system, in accordance with an embodiment.
Fig. 3 is a flowchart of a method for obtaining ultrasound images and user input, in accordance with an embodiment.
Fig. 4 is a flowchart of a method for processing multiple inputs to a localization algorithm, in accordance with an embodiment.
Fig. 5 is a flowchart of a method for providing localization information using an ultrasound system, in accordance with an embodiment.
Fig. 6 is a flowchart of a method for providing localization information using an ultrasound system, in accordance with an embodiment.
Fig. 7 is a flowchart of a method for providing localization information using an ultrasound system, in accordance with an embodiment.
Fig. 8 is a flowchart of a method for training a localization algorithm, in accordance with an embodiment.
Fig. 9 is a flowchart of a method for modifying a localization report, in accordance with an embodiment.
Fig. 10 is a schematic representation of a localization report, in accordance with an embodiment.

### DETAILED DESCRIPTION OF EMBODIMENTS

The present disclosure describes various embodiments of an ultrasound analysis system and method. More generally, Applicant has recognized and appreciated that it would be beneficial to provide an ultrasound analysis that provides improved feature localization or identification using a trained localization algorithm receiving both user input and ultrasound imaging as input. For example, an ultrasound analysis system receives or obtains a plurality of ultrasound images for a first imaged region. The system also receives, via a user interface, a user input comprising location information about the first imaged region. A trained localization algorithm of the system processes both: (i) one or more of the received plurality of ultrasound images; and (ii) the received user input to generate a localization report for the first imaged region, the localization report comprising one or more of a coordinate, a contour, and a structure identification. The system them provides the localization report to a user via a user interface. According to an embodiment, therefore, is a workflow, algorithm, and associated user interface aimed at facilitating faster ultrasound-based diagnostic and interventional imaging procedures. One example where the invention may be useful is to assist in peripheral artery disease (PAD) examinations, where ultrasound is widely used to measure lumen diameter, evaluate hemodynamic function, and support treatment decisions.

According to an embodiment, **ultrasound imaging workflows are simplified by assisting users with the quick localization and tracking of structures that would otherwise be challenging to localize. This could include automatically segmenting the boundaries of small arteries during ultrasound scans, as well as many other ultrasound imaging procedures. A key clinical benefit is to enable rapid interrogation of small, challenging structures, as well as more efficient quantification of relevant anatomical measurements imaged under ultrasound, by leveraging user and other input information to support image-based localization.**

The ultrasound analysis system and method disclosed or otherwise envisioned herein provides numerous advantages over the prior art. Providing an ultrasound analysis system and method that enables the improved localization and/or identification of landmarks or anatomical structures using both ultrasound images and user input can improve ultrasound analysis and/or diagnosis, improve patient outcomes, and potentially save lives.

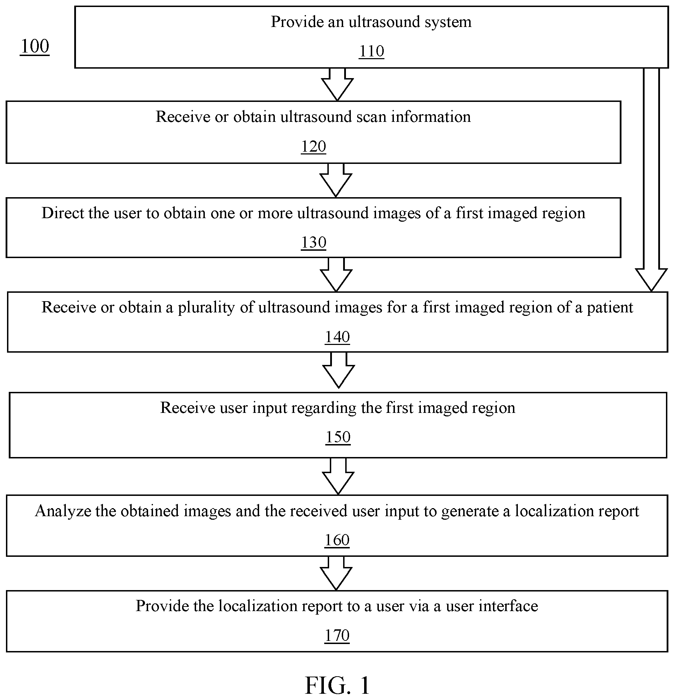

Referring to Fig. 1, in one embodiment, is a flowchart of a method 100 for providing localization information using an ultrasound system. The methods described in connection with the figures are provided as examples only, and shall be understood not to limit the scope of the disclosure. The ultrasound system can be any of the systems described or otherwise envisioned herein. The ultrasound system can be a single system or multiple different systems.

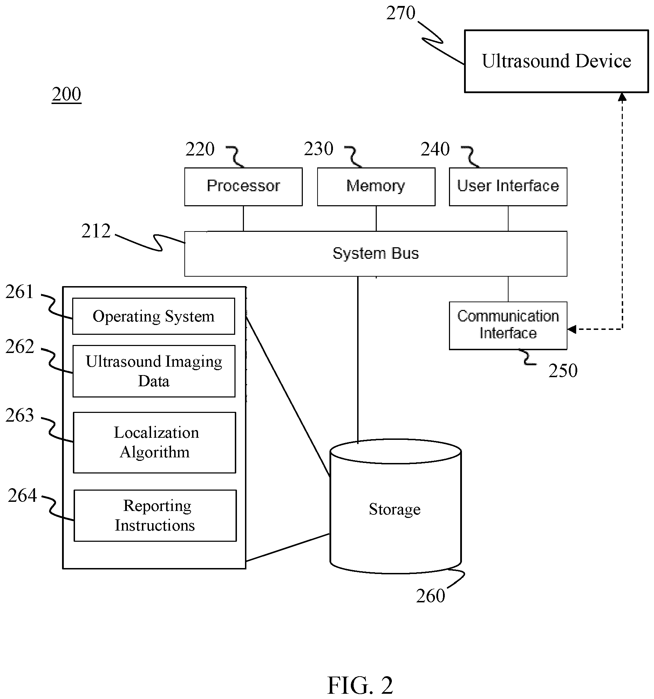

At step 110 of the method, an ultrasound system 200 is provided. Referring to an embodiment of an ultrasound system 200 as depicted in Fig. 2, for example, the system comprises one or more of a processor 220, memory 230, user interface 240, communications interface 250, storage 260, and ultrasound device 270, interconnected via one or more system buses 212. It will be understood that Fig. 2 constitutes, in some respects, an abstraction and that the actual organization of the components of the system 200 may be different and more complex than illustrated. Additionally, ultrasound system 200 can be any of the systems described or otherwise envisioned herein. Other elements and components of system 200 are disclosed and/or envisioned elsewhere herein.

At optional step 120 of the method, the ultrasound system obtains or receives ultrasound scan information for a present or future scan. The ultrasound scan information can be any information relevant to the plan, setting, region, or other aspect of a scan. For example, the system first detects or receives information about a particular scan (*e.g.,* a vascular scan) such as via a user pre-set selection. This information can be received via a user interface, or can be determined automatically by the system upon receiving or obtaining a setting, parameter, medical order, or other information about a present or future scan.

At optional step 130 of the method, the ultrasound system directs the user to obtain one or more ultrasound images of a first imaged region, based at least in part on the ultrasound scan information received or obtained by the ultrasound system. For example, the user interface can direct the user to acquire an ultrasound image (*e.g.,* a B-mode image) in the vicinity of the structure of interest (*e.g.,* an artery), its upstream branch, and/or its downstream branch. This instruction can be a user interface (UI) floating window that persists till the desired view is achieved, although other methods of providing instructions are possible.

At step 140 of the method, the ultrasound system receives or obtains a plurality of images for a first imaged region of a patient. According to an embodiment, ultrasound image data is sent to, obtained by, or otherwise received by the system. The ultrasound image data may comprise, for example, a temporal sequence of ultrasound image data such as a video comprising a plurality of frames. Ultrasound image data may be obtained for a single imaged region, or may be obtained for a plurality of regions. The imaged region may be any portion of a person or animal's body for which ultrasound data can be obtained. The ultrasound image data may be received by the system in real-time, or may be stored in local and/or remote memory and received by the system at a future point.

The ultrasound image data may be obtained using any ultrasound device or system, which may be any device or system suitable to obtain or otherwise receive ultrasound image data of the patient. One or more parameters of the ultrasound device can be set, adjusted, preprogrammed, or otherwise determined by a healthcare professional. The ultrasound device or system may be remote to, local to, or a component of, the ultrasound analysis system 200.

At step 150 of the method, the ultrasound system receives a user input comprising location information about the first imaged region. This user input, also referred to as a secondary input, can be received directly from a user via a user interface, or indirectly from the user by analysis of user action.

According to an embodiment, the user may select - such as upon instruction from or direction by the ultrasound system - a location of a landmark or anatomical feature/structure or another target in an image. For example, the user may select a landmark or anatomical feature/structure or other target in an image by clicking, tapping, or tracing a contour around the structure(s) that he/she is interested in scanning and analyzing during the procedure. According to an embodiment, the clicked/tapped position is converted to a binary or grayscale image mask, in which the pixels around the clicks can be marked as the foreground in the mask. For example, in a case where the ultrasound scan is being performed in color/power Doppler mode, the system may direct the user to position the Doppler box over the target(s) following standard scanning procedures. The coordinates of the center of the Doppler box (or alternatively, the four corners of the Doppler box) would be converted to an image mask. Thus, according to an embodiment, the user input may comprise a direct user action such as a mouse click to indicate a target (x, y) coordinate. According to an embodiment, the user input may be derived from the user's interactions with the ultrasound system. For example, the system may derive user input from actions such as the user positioning of the color Doppler box, a spectral Doppler gating window on B-mode images, user placement of focal depth markers, user-defined scan depth, user centering of the probe/image over the target, and/or the user performing probe compression (or other) maneuvers, among other user interactions.

According to an embodiment, the user input may be derived from the obtained ultrasound data, with or without direct user input. For example, the user input may be derived from color/power Doppler signals acquired alongside B-mode images, images collected previously on the same anatomy, algorithm predictions from the previous frame of the sequence and converted into location information, and/or other modes (such as elasticity or strain) to derive location information that can complement the B-mode imagery, among other obtained ultrasound data.

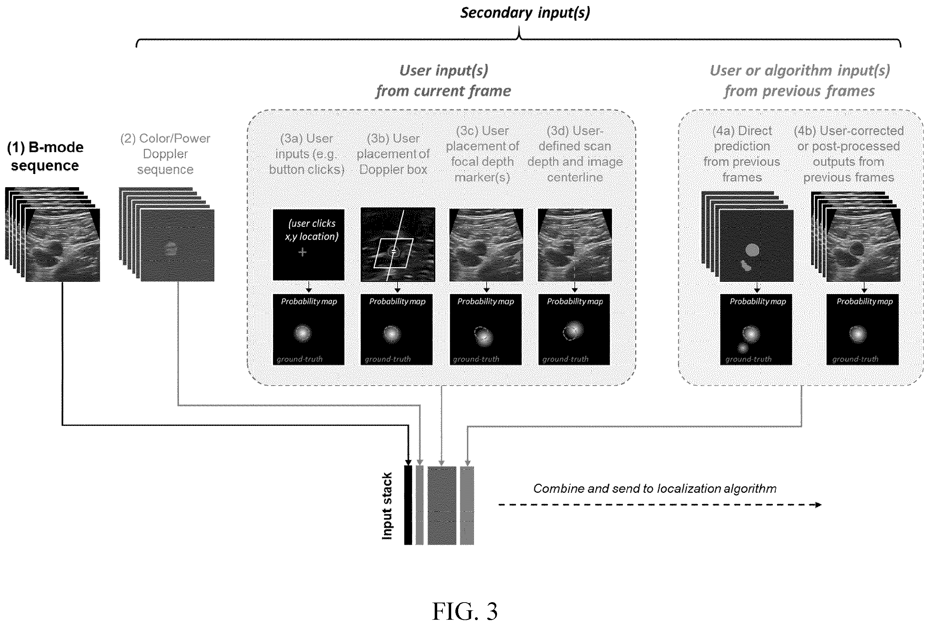

According to an embodiment, in order to utilize the user input, the system converts the user input into a format that the downstream localization algorithm can process. For example, the system can convert all input data into the form of image masks (such as binary or grayscale), in which the pixels around the location coordinates are marked as foreground in the mask. Referring to Fig. 3, in one embodiment, is a flowchart of a method for obtaining ultrasound images and user input to be utilized as input for a localization algorithm of the ultrasound system. In this embodiment, all secondary inputs are converted to smoothed grayscale Gaussian masks, with the Gaussian peak centered on the location coordinate indicating the location of maximum probability. In Fig. 3, multiple types of user inputs may be used by the localization algorithm, in addition to the ultrasound imaging (such as B-mode imaging). These secondary inputs could be other ultrasound imaging modes, such as color/power Doppler, user inputs from the current frame, and/or user or algorithm inputs from previous frames. The inputs are then combined (such as by concatenating all masks into a single input stack) and fed into the localization algorithm.

Thus, according to an embodiment, the input data is combined and fed into the localization algorithm such as by concatenating the masks (along with the B-mode frame) right at the beginning (i.e., early merging), as shown in Fig. 3. Alternatively, each input could be sent to a parallel channel of the network and undergo its own series of processing (convolutions) before being merged inside the network (i.e., late merging).

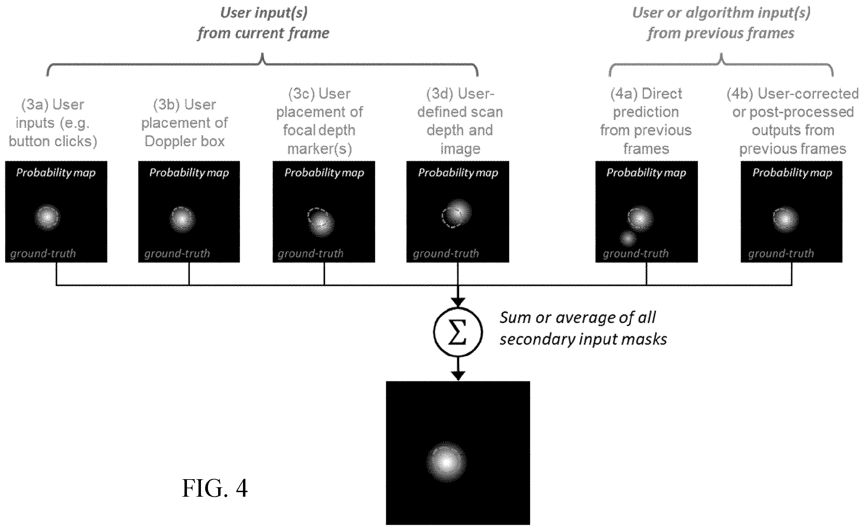

According to another embodiment, all the secondary masks are summed or averaged together to arrive at a single overall mask, which is sent into the localization algorithm, as shown in Fig. 4. In this approach, rather than concatenating inputs into a stack, individual masks (probability maps) are summed or averaged together to arrive at a single overall mask, resulting in a smaller/simpler algorithm or network for localization. One advantage of this approach is reducing the amount of processing required by the localization algorithm, allowing for a smaller/simpler network.

The ultrasound system can therefore comprise a user interface that allows user interaction data to be fed directly into the system as inputs. For example, the user can select targets of interest such as by clicking on a target. Thus, the user interface may include one or more devices for enabling communication with a user. The user interface can be any device or system that allows information to be conveyed and/or received, and may include a display, a mouse, and/or a keyboard for receiving user commands.

In addition to direct user inputs, the UI can support one or more ultrasound system controls that could be adjusted by the user and from which the algorithm can indirectly derive location information. One example, particularly relevant for vessel localization, is user positioning of the color Doppler box. For instance, the center of the color box may be the secondary location coordinate input into the localization algorithm. Or similarly, the four corners of the color box can be the location input. Another example is the scan depth and focal depth set by the user when scanning, since these are typically set to match the depth of the target structure(s). A third example is the center of the image laterally since the user will typically aim to center the ultrasound probe over the target structure(s). A fourth example is user positioning of the spectral Doppler gating window. Within this mode, user defines a small area (gate) within the B-mode image. The center and size of the gate can be converted as a location input to the algorithm. A fifth example is an elasticity/strain map to initialize possible locations. The system temporarily transitions to the appropriate elasticity mode for a few frames, gets the initialization data it needs (the elasticity/strain maps can be binarized using thresholding to form a direct input to the network) and then transitions back to B-mode to use that data in the algorithm. A sixth example can be a compression maneuver performed by the user, to differentiate, say, between a vein and an artery (a vein collapses upon compression, while an artery does not). As the user performs the probe compression, the system analyzes the US frames to automatically gather that knowledge. A separate in-built algorithm can look for changes in image pixel values during the compression maneuver.

According to an embodiment, any of this user input information could be provided once at the beginning of the scan, and/or updated one or more times during the scan to aid in tracking.

According to an embodiment, the user interface also displays the results of the algorithm (e.g., predicted locations/contours) in real time. The UI allows manual adjustment of predictions. The adjusted results are used as corrected input data for subsequent frames, or re-processing preceding frames during retrospective review. Again, this could be done once or multiple times throughout the scan.

The user interface can be used to reposition color/power/spectral Doppler boxes or adjust other settings such as the scan depth and focal depth. According to an embodiment, the user interface can be used to make measurements during the scan. For example, as the user scans along the leg, the user may be interested in measuring vessel diameters or stenoses at specific locations. A screen tap or button press would trigger a separate algorithm to calculate diameter/area/other measurements within segmented vessels in the current frame. The UI may further prompt the user to make measurements in specific anatomical areas. For example, when a bifurcation is detected based on the segmentation output, the system beeps or shows an indicator on the screen that suggests to the user to make a measurement in that FOV. When the user stops moving the probe (or freezes the image), the system can then proceed to make (or initialize) those measurements as described herein.

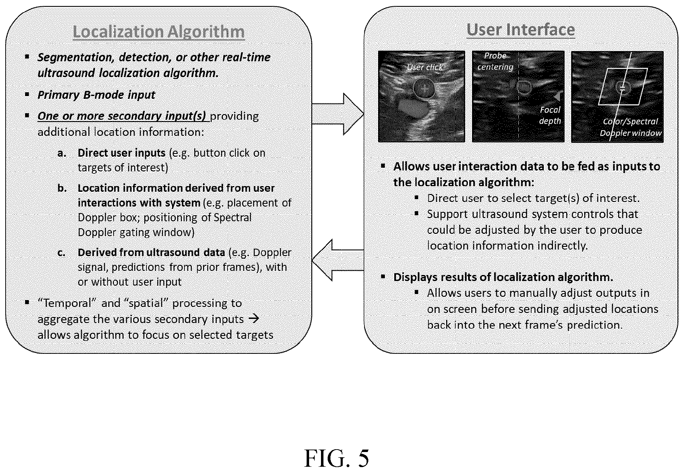

At step 160 of the method, a trained localization algorithm processes both one or more of the received plurality of ultrasound images, and the received user input, as input. The trained localization algorithm utilizes this input to generate a localization report for the first imaged region, where the localization report includes one or more of a coordinate, a contour, and a structure identification. For example, referring to Fig. 5, in one embodiment, is a schematic representation of the ultrasound system workflow for localization. The algorithm processes a sequence of ultrasound images along with one or more secondary inputs that give additional location information. The interface allows secondary inputs to be generated, including from user inputs and user interactions with the system.

According to an embodiment, the algorithm may be a segmentation algorithm, object detection algorithm, or other localization algorithm. The algorithm may be a deep neural network model that includes mechanisms for incorporating multiple different inputs, including user-derived inputs, although other algorithms are possible. The algorithm can be capable of temporal and/or spatial processing to aggregate the various forms of secondary input data. This allows the algorithm to focus on selected target(s). According to an embodiment, the algorithm outputs the coordinates and/or contours of detected structures in each frame of the ultrasound sequence. A confidence score can be produced and provided with each prediction.

Notably, according to an embodiment, not all weights in the neural works are fixated. For example, spatial attention gates can be used to generate attention weights dynamically based on the image context. According to an embodiment, the system can include a specific UI panel that appears at the beginning of the scan to allow the user to input all relevant location information (user clicks, Doppler box placement, scan/focal depth, etc.). Multiple forms of location information may be provided in this manner to initialize the localization algorithm.

According to an embodiment, the system can operate with color/power Doppler turned on. The Doppler image can be a second data input, which could similarly be concatenated with the B-mode and mask images. As mentioned above, when Doppler is turned on, the position of the Doppler box serves as one of the secondary user-derived inputs to the algorithm.

According to an embodiment, the localization algorithm allows temporal and spatial processing to aggregate various forms of secondary input data. For example, rather than operating on frames independently, the algorithm can maintain a memory of prior data. This is critical to be able to track the target(s) identified in the initial frame throughout the scan. The prior data may be user input information, previous B-mode/ Doppler views, previous model predictions from earlier frames, or a combination of these. In any case, the prior data is converted to location information as part of the secondary input. The prior information can be updated continuously as the scan progresses based on the new incoming frame information. The user may interact with the prior information maintained in memory. For instance, if the user adjusts the location/contours predicted by the localization algorithm, the adjusted results used for subsequent frame predictions to improve tracking accuracy.

Similarly, the user may direct the system to store particular views that they like during the scan. These could for example be frames with particularly high image quality or where the algorithm produces highly accurate results. These snapshot views (or the location information derived at these specific frames) become subsequent secondary inputs into the localization algorithm.

As part of being able to remember prior data, the localization algorithm can operate on time series (i.e., video sequences) as opposed to single ultrasound frames. For example, the algorithm can include a recurrent neural network architecture that introduces temporal units to achieve temporal awareness. These units provide a mechanism to encode memory from prior frames and feed this information part of the secondary input subsequent frame predictions.

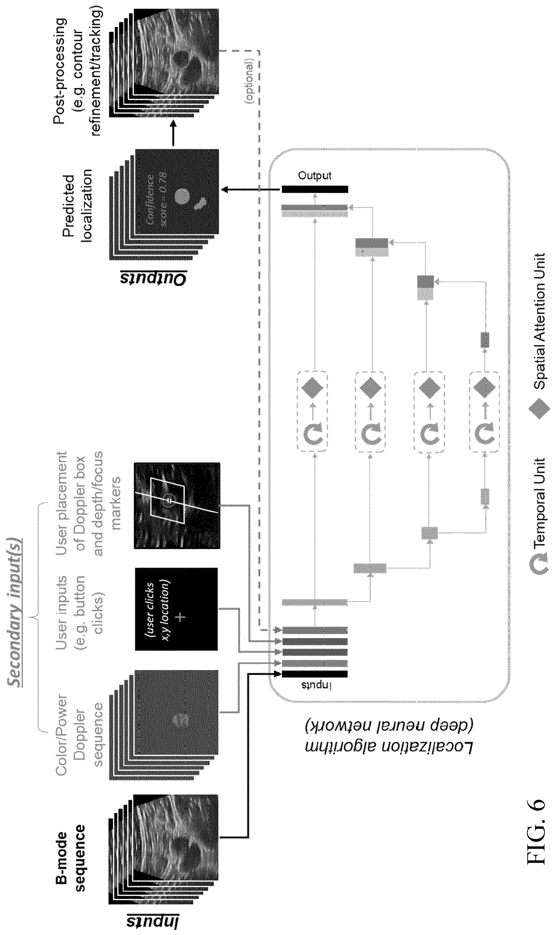

Referring to Fig. 6, in one embodiment, is a flowchart showing one way to incorporate temporal memory into a deep learning model. In this embodiment, the localization algorithm is a deep neural network that takes B-mode images and a set of secondary input(s). A segmentation network is shown, but the system is not limited to a particular type of network. The example is artery localization, but the system may be applied to other real-time ultrasound imaging use cases.

According to an embodiment, the localization algorithm incorporates a mechanism to maintain global spatial information about the image, specifically by aggregating spatial information throughout all pixels of the image and encoding global image context. Since all of the secondary inputs come in the form of location information, efficient aggregation of spatial information is critical. This can be this is achieved by introducing spatial attention units into the proposed neural network. Feasibility experiments indicate that these units result in more accurate segmentation of very small structures, which otherwise might be buried within the large number of background pixels in the frame. Fig. 6 depicts one mechanism to incorporate spatial attention into a model.

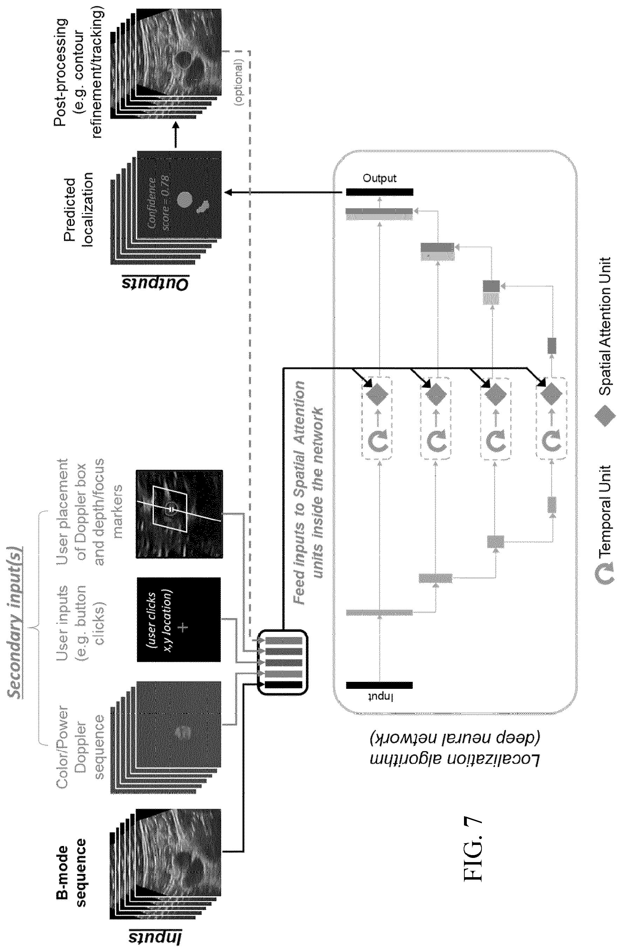

According to an embodiment, an added potential function of the Spatial Attention Units is to directly receive the various secondary location information. Referring to Fig. 7, in one embodiment, is a flowchart of an implementation of the localization algorithm. In contrast to the embodiment in Fig. 6, the inputs are passed to each of the individual' Spatial Attention Units' in the network, rather than passed in at the beginning of the network. The spatial attention units are naturally designed to encode position information. Since the Spatial Attention Units are naturally designed to encode position information, the location data may be fed directly to these units rather than being sent in at the beginning of the network (i.e. alongside the full B-mode frame). A potential advantage of this approach is that, since there are multiple Spatial Attention Units distributed across the layers of the network, it allows more ways to introduce the location information throughout the network, including at multiple spatial resolution scales. At step 170 of the method, the ultrasound system provides the generated localization report to the user via a user interface. The localization report may comprise one or more of **a coordinate, a contour, and a structure identification, among other information.** The localization report can be provided via the user interface using any method for conveying or displaying information, and the user interface can be any device, interface, or mechanism for providing the conveyed or displayed information. According to an embodiment, the localization report may be communicated by wired and/or wireless communication to another device. For example, the system may communicate the report to a mobile phone, computer, laptop, wearable device, and/or any other device configured to allow display and/or other communication of the report.

**According to an embodiment, a user interface** displays the outputs of the localization algorithm. These may be markings/contours on the screen, highlighted annotations (e.g., regions of narrowing or bifurcation), user guidance elements (e.g., left/right arrows to guide the scan), updated regions-of-interest (e.g., for automated Doppler box placement), display of confidence/quality scores, and/or quantitative measurements derived from the localization predictions, among other features. **According to an embodiment, as a user is** scanning, the UI allows the user to make live adjustments or correction of the detected location/contours produced by the localization algorithm. If the user edits the contour on one frame, the manually adjusted results are then used in place of the direct algorithm output for subsequent frame predictions to improve tracking.

According to an embodiment, localization algorithm outputs in real-time the location of detected targets. Additional outputs could include a segmentation mask or a set of coordinates (bounding box, centroid coordinates, radius, eclipse fitting, etc.). The algorithm may also produce a set of secondary outputs from the temporal and spatial attention units. The outputs are then used as inputs for prediction of the next frame.

The localization algorithm may additionally output a confidence or quality score to indicate the algorithm's confidence in its prediction. For instance, if the targets are segmented poorly as determined by the confidence score, the system warns the user that the target is lost (i.e., the confidence score is below a threshold). All primary or secondary outputs from the algorithm may be interacted with by the user and be updated in this manner prior to being sent back in as inputs in subsequent frames to improve tracking accuracy during the scan.

According to an embodiment, post-processing may be applied to each prediction to refine the predicted vessel contours in each frame, clean up noisy segmentation regions, and/or perform frame-to-frame tracking of the vessels. The post-processing approach could be a spline adaptation, active-contours propagation, Kalman/particle filter tracking, or other methods based on classical image processing. The post-processing could also involve user adjustments. If any post-processing is applied, the updated results are used as input for the next frame prediction instead of the raw predictions.

According to an embodiment, sudden changes in the shape or trajectory of the target structures may be detected directly from the algorithm's outputs. Most importantly for the vascular clinical application, this includes vessel bifurcations (when a vessel splits into two or joins into one) and sudden vessel narrowing or flattening (decrease in vessel diameter or cross-sectional area). These events are highlighted to the user so they are aware of changes and can choose to modify the scan or manually adjust the predicted outputs.

Other localization tasks, such as object detection or pose regression, can make use of the above methods and embodiments.

Thus, according to an embodiment, the UI directs the user to sweep the probe along the target structure, and the algorithm maintains a memory of past views to track the target detected in the initial frame while updating continuously. The detections can be updated continuously throughout the sweep. For each frame collected, the output is the location of detected target structures, a binary segmentation mask of detected target structures, and a secondary input to the prediction of the next frame. Post-processing may be applied to refine the network's outputs, remove noisy regions, and propagate predicted contours to the next frame.

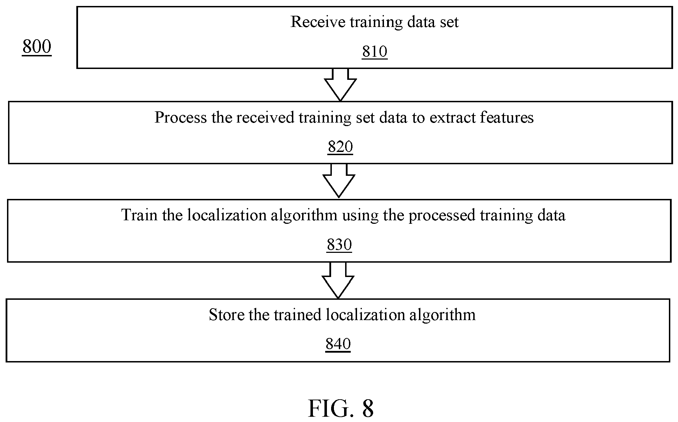

Referring to Fig. 8, in one embodiment, is a flowchart of a method 800 **for training the localization algorithm of the ultrasound system. At step 810 of the method, the system receives a training data set comprising ultrasound data, preferably for a plurality of ultrasound scans for a plurality of imaged regions and a plurality of** patients. The training data can comprise any information or data necessary to train the localization algorithm, including labeled ultrasound data where structures or anatomical features are labeled or predicted, and secondary data such as user input (including any of the forms of user input described or otherwise envisioned herein) provides additional information about the structures or anatomical features. The training data may be stored in and/or received from one or more databases. The database may be a local and/or remote database. For example, the ultrasound system may comprise a database of training data.

According to an embodiment, the ultrasound system may comprise a data pre-processor or similar component or algorithm configured to process the received training data. For example, the data pre-processor analyzes the training data to remove noise, bias, errors, and other potential issues. The data pre-processor may also analyze the input data to remove low quality data. Many other forms of data preprocessing or data point identification and/or extraction are possible.

At step 820 of the method, the system processes the received information to extract localization features about the ultrasound data. The localization features may be any features which will be utilized to train the localization algorithm, such as any localization features that can or will be utilized by the trained algorithm for localization analysis for a future ultrasound scan. Feature extraction can be accomplished by a variety of embodiments for feature identification, extraction, and/or processing, including any method for extracting features from a dataset. The outcome of a feature processing step or module of the ultrasound system is a set of localization features about a **plurality of ultrasound scans for a plurality of imaged regions and a plurality of** patients, which thus comprises a training data set that can be utilized to train the classifier.

At step 830 of the method, the system trains the machine learning algorithm, which will be the algorithm utilized in analyzing ultrasound information as described or otherwise envisioned. The machine learning algorithm is trained using the extracted features according to known methods for training a machine learning algorithm. According to an embodiment, the algorithm is trained, **using the processed training dataset, to generate a localization report based on ultrasound images and user input, as described or otherwise envisioned herein. The generated localization report can comprise any of the information described or otherwise envisioned herein, including but not limited to identification or localization of anatomical structures, confidence scores, and other information.**

**At step 840 of the method, the trained** localization algorithm **is stored for future use. According to an embodiment, the trained model may be stored in local or remote storage.**

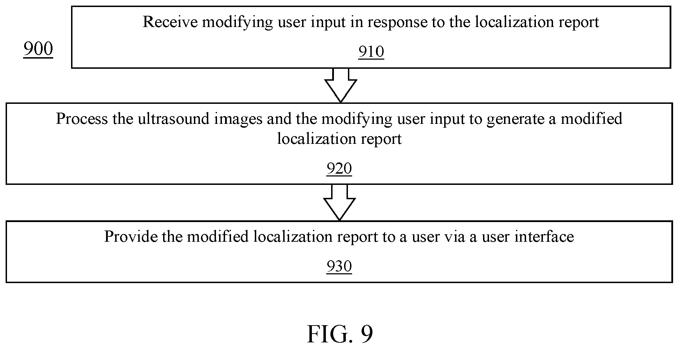

**Referring to** **Fig. 9****, in one embodiment, is a flowchart of a method for modifying the information provided in a localization report generated by the method of** **Fig. 1****. At step 910 of the method, the ultrasound system receives,** from the user via the user interface in response to the provided localization report, modifying user input about the first imaged region. For example, the user may review the provided localization report and identify an issue that can be fixed, modified, or otherwise adjusted in the algorithmic analysis. The modifying user input can be provided using any of the methods for providing user input described or otherwise envisioned herein.

At step 920, the trained localization algorithm processes the ultrasound images and the modifying user input to generate a modified localization report for the first imaged region. The trained localization algorithm processes the input using any of the methods described or otherwise envisioned herein. The modified localization report can comprise any of the information described or otherwise envisioned herein.

At step 930, the system provides the modified to a user via the user interface. The localization report can be provided via the user interface using any method for conveying or displaying information, and the user interface can be any device, interface, or mechanism for providing the conveyed or displayed information. According to an embodiment, the localization report may be communicated by wired and/or wireless communication to another device. For example, the system may communicate the report to a mobile phone, computer, laptop, wearable device, and/or any other device configured to allow display and/or other communication of the report.

Referring to Fig. 2, in one embodiment, is a schematic representation of an ultrasound system 200. System 200 may be any of the systems described or otherwise envisioned herein, and may comprise any of the components described or otherwise envisioned herein. It will be understood that Fig. 2 constitutes, in some respects, an abstraction and that the actual organization of the components of the system 200 may be different and more complex than illustrated.

According to an embodiment, system 200 comprises a processor 220 capable of executing instructions stored in memory 230 or storage 260 or otherwise processing data to, for example, perform one or more steps of the method. Processor 220 may be formed of one or multiple modules. Processor 220 may take any suitable form, including but not limited to a microprocessor, microcontroller, multiple microcontrollers, circuitry, field programmable gate array (FPGA), application-specific integrated circuit (ASIC), a single processor, or plural processors.

Memory 230 can take any suitable form, including a non-volatile memory and/or RAM. The memory 230 may include various memories such as, for example L1, L2, or L3 cache or system memory. As such, the memory 230 may include static random access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 200. It will be apparent that, in embodiments where the processor implements one or more of the functions described herein in hardware, the software described as corresponding to such functionality in other embodiments may be omitted. User interface 240 may include one or more devices for enabling communication with a user. The user interface can be any device or system that allows information to be conveyed and/or received, and may include a display, a mouse, and/or a keyboard for receiving user commands. In some embodiments, user interface 240 may include a command line interface or graphical user interface that may be presented to a remote terminal via communication interface 250. The user interface may be located with one or more other components of the system, or may be located remote from the system and in communication via a wired and/or wireless communications network.

Communication interface 250 may include one or more devices for enabling communication with other hardware devices. For example, communication interface 250 may include a network interface card (NIC) configured to communicate according to the Ethernet protocol. Additionally, communication interface 250 may implement a TCP/IP stack for communication according to the TCP/IP protocols. Various alternative or additional hardware or configurations for communication interface 250 will be apparent. Storage 260 may include one or more machine-readable storage media such as read-only memory (ROM), random-access memory (RAM), magnetic disk storage media, optical storage media, flash-memory devices, or similar storage media. In various embodiments, storage 260 may store instructions for execution by processor 220 or data upon which processor 220 may operate. For example, storage 260 may store an operating system 261 for controlling various operations of system 200.

It will be apparent that various information described as stored in storage 260 may be additionally or alternatively stored in memory 230. In this respect, memory 230 may also be considered to constitute a storage device and storage 260 may be considered a memory. Various other arrangements will be apparent. Further, memory 230 and storage 260 may both be considered to be non-transitory machine-readable media. As used herein, the term non-transitory will be understood to exclude transitory signals but to include all forms of storage, including both volatile and non-volatile memories.

While system 200 is shown as including one of each described component, the various components may be duplicated in various embodiments. For example, processor 220 may include multiple microprocessors that are configured to independently execute the methods described herein or are configured to perform steps or subroutines of the methods described herein such that the multiple processors cooperate to achieve the functionality described herein. Further, where one or more components of system 200 is implemented in a cloud computing system, the various hardware components may belong to separate physical systems. For example, processor 220 may include a first processor in a first server and a second processor in a second server. Many other variations and configurations are possible.

According to an embodiment, storage 260 of system 200 may store one or more algorithms, modules, and/or instructions to carry out one or more functions or steps of the methods described or otherwise envisioned herein. For example, the system may comprise, among other instructions or data, ultrasound imaging data 262, localization algorithm 263, and/or reporting instructions 264, among many other possible instructions and/or data.

According to an embodiment, ultrasound imaging data 262 is any ultrasound imaging data that is sent to, obtained by, or otherwise received by the system. The ultrasound image data may comprise, for example, a temporal sequence of ultrasound image data such as a video comprising a plurality of frames. Ultrasound image data may be obtained for a single region or may be obtained for a plurality of different regions. The ultrasound image data may be obtained using any ultrasound device or system, which may be any device or system suitable to obtain or otherwise receive ultrasound image data of the patient. The ultrasound device or system may be remote to, local to, or a component of, the ultrasound analysis system 200.

According to an embodiment, localization algorithm 263 is any model or algorithm that is trained or configured to utilize both: (i) one or more of the received plurality of ultrasound images; and (ii) the received user input, in order to generate a localization report for the first imaged region. The trained localization algorithm processes the input using any of the methods described or otherwise envisioned herein. The localization report can comprise any of the information described or otherwise envisioned herein.

According to an embodiment, reporting instructions 264 direct the system to generate and provide a report or visualization to a user via the user interface 240 of the ultrasound system 200. The localization report can comprise any of the information described or otherwise envisioned herein. Other information is possible as well, including but not limited to the identity of the patient, patient demographics, diagnosis or treatment information, and a wide variety of other possible information. The information can be provided via the user interface using any method for conveying or displaying information, and the user interface can be any device, interface, or mechanism for providing the conveyed or displayed information. According to an embodiment, the instructions may direct the system to display the information on the user interface or display of the system. The report may be communicated by wired and/or wireless communication to another device. For example, the system may communicate the report to a mobile phone, computer, laptop, wearable device, and/or any other device configured to allow display and/or other communication of the report.

### EXAMPLE

The following is a non-limiting example of the utilization of a localization algorithm as described or otherwise envisioned herein for vessel segmentation.

According to an embodiment, the methods and systems described herein were implemented as shown in Fig. 6. The model takes the B-mode video sequence, color Doppler video sequence, and user input masks (button click of user-selected vessels converted to binary masks) as a set of concatenated multi-channel inputs. The primary output is a segmentation mask of same size as the input images that is tracked throughout the scan. A localization confidence/quality score is also produced for each object in each frame.

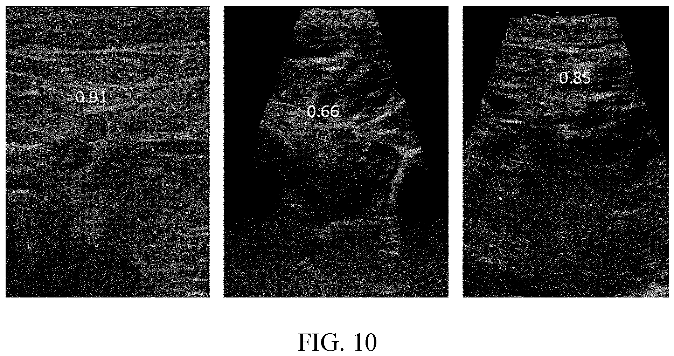

The user interface is important to the overall invention, as it allows the user to select target vessels to track and displays automated vessel diameter measurements and quality/confidence scores. Results of the trained network are shown in Fig. 10, showing vessel segmentations produced by the localization algorithm. From left to right: femoral artery (6 mm diameter), anterior tibial artery (2 mm diameter), and posterior tibial artery (3 mm diameter). Confidence scores are calculated by taking the average of scores for all pixels within the segmented vessels.

These results illustrate the ability of the proposed algorithm to segment different peripheral vessels with a reasonable degree of confidence. The anterior and posterior tibial arteries (ATA and PTA) are among the smallest arteries in the peripheral vasculature that routinely have to be imaged to evaluate peripheral vascular health. The impact of the secondary inputs, including simulated user-click inputs during the scan sequence, is highlighted in feasibility experiments carried out on ultrasound sequences acquired during lower extremity scans on healthy subjects (TABLE 1), where the tibial arteries-of-interest represented <0.1% of the pixels in each frame (2-3 mm diameter). The baseline algorithm is a U-Net deep learning segmentation model trained on small tibial arteries (2-3 mm diameter) from nine exams acquired on healthy subjects. Results are on independent exams unseen by the model. The impact of the secondary inputs, including simulated user-click inputs during the scan sequence, is highlighted.

As mentioned, the methods and systems are not limited to segmentation nor to a particular network architecture. Other localization tasks, such as object detection or pose regression, can also make use of the proposed methods.

**TABLE 1**

| Model | B-mode | Doppler | Spatial + temporal attention | User input (simulated user clicks during scan sequence) | Dice score (mean ± stdv) |
|---|---|---|---|---|---|
| Baseline + Doppler | X | X | | | 0.527 ± 0.336 |
| Baseline + Doppler + Spatiotemporal | X | X | X | | 0.671 ± 0.240 |
| Baseline + Doppler + Spatiotemporal + User Input Clicks | X | X | X | X | 0.751 ± 0.174 |

According to an embodiment, the ultrasound system is configured to process many thousands or millions of datapoints in the input data used to train the classifier, as well as to process and analyze the received plurality of localization features. For example, generating a functional and skilled trained classifier using an automated process such as feature identification and extraction and subsequent training requires processing of millions of datapoints from input data and the generated features. This can require millions or billions of calculations to generate a novel trained classifier from those millions of datapoints and millions or billions of calculations. As a result, each trained classifier is novel and distinct based on the input data and parameters of the machine learning algorithm, and thus improves the functioning of the ultrasound system. Thus, generating a functional and skilled trained classifier comprises a process with a volume of calculation and analysis that a human brain cannot accomplish in a lifetime, or multiple lifetimes.

In addition, the ultrasound system can be configured to continually receive localization features, perform the analysis, and provide periodic or continual updates via the report provided to a user for the patient. This requires the analysis of thousands or millions of datapoints on a continual basis to optimize the reporting, requiring a volume of calculation and analysis that a human brain cannot accomplish in a lifetime.

By providing an improved ultrasound analysis, this novel ultrasound system has an enormous positive effect on patient care compared to prior art systems. As just one example in a clinical setting, by providing a system that can improve patient analysis and diagnosis with confidence intervals, the system can facilitate treatment decisions and improve survival outcomes, thereby leading to saved lives.

The present invention may be a system, a method, and/or a computer program product. The computer program product may include a non-transitory computer readable storage medium (or media) having computer readable program instructions thereon for causing a system or processor to carry out aspects of the present invention. The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any combination of the foregoing, among other possibilities. Computer readable program instructions described herein can be downloaded to respective computing/processing devices from a computer readable storage medium or to an external computer or external storage device via a network, for example, the internet, a local area network, and/or a wireless network. Aspects of the present invention are described herein with reference to flowchart illustrations and/or block diagrams of methods, apparatus, systems, and computer program products according to embodiments of the invention. It will be understood that each block of the flowchart illustrations and/or block diagrams, and combinations of blocks in the flowchart illustrations and/or block diagrams, can be implemented by computer readable program instructions.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively, as set forth in the United States Patent Office Manual of Patent Examining Procedures, Section 2111.03.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method (100) for providing localization information using an ultrasound system (200), comprising:
receiving (140) a plurality of ultrasound images for a first imaged region;
receiving (150) an input comprising location information about the first imaged region;
processing (160), using a trained localization algorithm, both: (i) one or more of the received plurality of ultrasound images and (ii) the received input to generate a localization report for the first imaged region, the localization report comprising one or more of a coordinate, a contour, and a structure identification; and
providing (170), via a user interface, the localization report to a user.

2. The method of claim 1, further comprising the steps of:
receiving (120) ultrasound scan information for a present or future scan; and
directing (130), based on the received ultrasound scan information, the user to obtain the plurality of ultrasound images for the first imaged region.

3. The method of claim 1, wherein the plurality of ultrasound images are received and processed in real time.

4. The method of claim 1, wherein the trained localization algorithm is a segmentation algorithm or an object detection algorithm.

5. The method of claim 1, wherein the localization report further comprises a confidence score generated by the trained localization algorithm.

6. The method of claim 1, wherein the input is derived from at least one of: the user's interaction with the ultrasound system while the user obtains the plurality of ultrasound images for the first imaged region, and one or more ultrasound scans or ultrasound images obtained or received before the received plurality of ultrasound images.

7. The method of claim 1, wherein the input is an identification of a structure or feature within the first imaged region.

8. The method of claim 1, further comprising:
receiving (910), from the user via the user interface in response to the provided localization report, modifying input about the first imaged region;
processing (920), using the trained localization algorithm, both: (i) one or more of the received plurality of ultrasound images and (ii) the received modifying input to generate a modified localization report for the first imaged region; and
providing (930), via a user interface, the modified localization report to the user.

9. An ultrasound system (200) configured to provide localization information, comprising:
a plurality of ultrasound images (262) for a first imaged region;
a user interface (240) configured to receive a user input comprising location information about the first imaged region;
a trained localization algorithm (263);
a processor (220) configured to process, using the trained localization algorithm both: (i) one or more of the received plurality of ultrasound images and (ii) the received user input to generate a localization report for the first imaged region; and direct the user interface to provide the localization report to a user.

10. The ultrasound system of claim 9, wherein the localization report comprises one or more of a coordinate, a contour, and a structure identification.

11. The ultrasound system of claim 9, wherein the localization report further comprises a confidence score generated by the trained localization algorithm.

12. The ultrasound system of claim 9, wherein the user input is derived from the user's interaction with the ultrasound system while the user obtains the plurality of ultrasound images for the first imaged region.

13. The ultrasound system of claim 9, wherein the user input is derived from one or more ultrasound scans or ultrasound images obtained or received before the received plurality of ultrasound images.

14. The ultrasound system of claim 9, wherein the user input is an identification of a structure or feature within the first imaged region.

15. A non-transitory computer readable storage medium (260) having computer readable program code embodied therein for causing an ultrasound system (200) to provide localization information by:
receiving a plurality of ultrasound images for a first imaged region;
receiving an input comprising location information about the first imaged region;
processing, using a trained localization algorithm, both: (i) one or more of the received plurality of ultrasound images and (ii) the received input to generate a localization report for the first imaged region, the localization report comprising one or more of a coordinate, a contour, and a structure identification; and
providing, via a user interface, the localization report to a user.
